# EUROPEAN PATENT SPECIFICATION

(11) **EP 3 515 525 B1**
(45) Date of publication and mention of the grant of the patent: **29.11.2023**
(21) Application number: 17851205.9
(22) Date of filing: 08.03.2017
(51) Int. Cl.: A61M 60/178, A61M 60/232, A61M 60/422, A61M 60/585, A61M 60/829, A61M 60/857, A61M 60/859, A61M 60/861, A61M 60/88, A61M 39/10

(54) **HEART CANNULA**
HERZKANÜLE
CANULE CARDIAQUE

(30) Priority: 19.09.2016 US 201662396606 P
(43) Date of publication of application: 31.07.2019
(73) Proprietor: Evaheart, Inc., Houston, TX 77030 (US); Sun Medical Technology Research Corporation, 392-0012 Nagano Nagano (JP)
(72) Inventor: MOTOMURA, Tadashi, Houston, TX 77025 (US); BENKOWSKI, Robert Joseph, Fort Worth, TX 76109 (US); MIYAKOSHI, Takayuki, Houston, TX 77030 (US)
(74) Representative: Zanettin, Gianluigi
(86) International application number: PCT/US2017/021358
(87) International publication number: WO 2018/052482

(56) References cited:
- EP-A2- 1 462 133
- WO-A1-00/37139
- WO-A1-2011/081629
- WO-A1-2016/018434
- US-A- 3 553 736
- US-A1- 2002 095 210
- US-A1- 2003 023 255
- US-A1- 2011 118 829
- US-A1- 2013 012 761
- US-A1- 2014 100 430

## Description

### Cross-Reference to Related Application

This Application is being filed on March 8, 2017, as a PCT International application claiming the benefit of U.S. Provisional Patent Application No. 62/396,606, filed on September 19, 2016.

### Background

Various medical devices require connection to a patient's heart, and more particularly, require a connection that provides fluid communication with a chamber of the heart. For example, the class of cardiovascular medical products referred to as "mechanical circulatory support" (MCS), including blood pump systems such as Left Ventricular Assist Devices (LVAD), require a fluid connection to a heart chamber. An LVAD is a medical device for patients in heart failure to bridge these patients to cardiac transplantation ("bridge-to-transplant") or for longer-term, permanent use ("destination therapy"). An LVAD works by draining blood from the patient's poorlyfunctioning left ventricle and pumping the blood to the aorta. A blood inflow cannula functions as a conduit to drain the blood from the heart to the LVAD or other pumping device so that the blood may be pumped to the aorta. WO2011/081629A1 discloses an implantable blood pump system, comprising a cannula system according to the preamble of claim 1.

### Summary

In accordance with certain aspects of the disclosure, a cannula system for a heart includes a cannula having an end configured for connection to a myocardium of a heart. In some examples, the opposite end of the cannula is configured for connection to an inlet of a pump, such as a LVAD pump. The cannula defines an inlet and has a flange or other connector extending around the cannula. A first cuff extends around the cannula and covers a substantial portion of an exterior surface of the cannula between the flange and the inlet. According to the invention, the first cuff may cover at least half or a majority of the exterior surface of the cannula between the flange and the inlet. By so constructing the first end of the cannula, the inlet extends into the patient's heart chamber minimally or not at all. For instance, in some embodiments, the tip extends not more than 5 mm beyond the endocardium, and in other examples the tip extends not more than 0.5 mm beyond the endocardium into the heart chamber. Additionally, a second cuff may be included that extends around the first end of the cannula adjacent the second end of the cannula at a distal end of the first cuff.

### Brief Description of the Drawings

Figure 1 is a perspective view illustrating an example of a cannula system attached to an inlet of a blood pump.
Figure 2 conceptually illustrates the cannula system and blood pump shown in Figure 1 implanted in a human body and connected to a human heart by the cannula system.
Figure 3 is a section view illustrating an example of the blood pump shown in Figures 1 and 2.
Figure 4 is a schematic section view of an example of the cannula system shown in Figures 1 and 2, and a portion of the heart to which it is attached.
Figure 5 is a perspective view of a connection end of an example of the cannula system shown in Figures 1 and 2.
Figure 6 is a side sectional view of an example of the cannula system shown in Figures 1 and 2.
Figure 7A is a side view illustrating an example cannula of the cannula system shown in Figure 6.
Figure 7B is a section view of the example cannula taken along line B in Figure 7A
Figures 8A-8D illustrate aspects of example sewing cuffs of the cannula system disclosed herein.
Figures 9-16 illustrate portions of an example surgical procedure for attaching the disclosed cannula system to a heart.

### Detailed Description

In the following Detailed Description, reference is made to the accompanying drawings, which form a part hereof, and in which is shown by way of illustration specific embodiments in which the invention may be practiced. In this regard, directional terminology, such as top, bottom, front, back, etc., is used with reference to the orientation of the Figure(s) being described. Because components of embodiments can be positioned in a number of different orientations, the directional terminology is used for purposes of illustration and is in no way limiting. It is to be understood that other embodiments may be utilized and structural or logical changes may be made without departing from the scope of the present invention. The following detailed description, therefore, is not to be taken in a limiting sense.

Various types of medical devices require connection to a human heart. Procedures such as aortic valve bypass and various heart pump installations require a fluid connection to a chamber of a human heart. For example, a ventricular assist device (VAD) is an electromechanical device that partially or completely replaces the function of a failing heart. Some VADs are for short-term use, while others are for long-term use. VADs are designed to assist either the right ventricle (RVAD) or the left ventricle (LVAD), or to assist both ventricles (BiVAD). The type of ventricular assistance device applied depends upon many factors.

The LVAD is the most common device applied to an impaired left heart function, but the right heart function can be impaired as well. In such situations, an RVAD might be necessary to resolve the problem of cardiac circulation. Normally, the long-term VAD is used as a bridge to transplantation - keeping alive the patient, with a good quality of life - while awaiting a heart transplant. In other circumstances, an LVAD may be applied as a bridge to recovery, and in still other circumstances, the LVAD may be applied as a long-term or permanent solution, also known as Destination Therapy (DT).

In the case of an LVAD, blood is drained from the left ventricle and pumped to the aorta. An inflow cannula is used to connect the heart to the fluid input of the LVAD. The cannula is inserted through the myocardial wall and engaged from the outside of the heart by surgical sutures. In general, known LVAD inflow cannulae clinically include a tip structure long enough to penetrate the myocardial wall and protrude well into the ventricular chamber to ensure that the cannula ostium is patent for blood drainage. However, a narrow space between the cannula tip extending into the ventricular chamber and the myocardial wall can cause an abnormal blood flow pattern around the cannula resulting in undesirable effects such as blood stagnation, turbulent flow, high shear flow, and flow separation, which may lead to blood clotgeneration.

Also, the blood-contacting surface of the protruding cannula tip may be a source of blood clots and other vulnerable cell/fibrin deposition as a physiologic response to enhanced coagulation cascade. Blood clots tend to form around the cannula, such as "wedge thrombus" - thrombus developed at the gap between myocardium and cannula wall. If such blood clots are sucked into the LVAD, it could lead to strokes. Furthermore, cannula tip malposition in the left ventricle due to poor anatomical fitting of the LVAD-pump and cannula can enhance the blood clot issue. Moreover, the tilting cannula tip could also rub and abrade the septum if there is misalignment of the protruding tip. The tilting tip could also become occluded if impinging on the septum.

Attempts to mitigate problems outlined above have been largely insufficient. For instance, different shapes of cannula tips have been applied in an attempt to optimize blood flow patterns in the left ventricle, but there is no satisfactory solution to normalize the blood flow pattern in the left ventricle. Some known LVADs include a textured surface added to the smooth metal surface of the cannula. Titanium sintered beads are sometimes used as surface texturization for LVAD cannula, made out of pure titanium or titanium alloy. Titanium sintered beads permit or enhance the formation of a thin layer of blood clot and eventually neo-intimal tissue ingrowth can take place. Such cannula covered with neo-intimal tissue - or in other words, an endothelialized cannula surface - can demonstrate superior antithrombogenicity. However, according to the recent clinical reports, even if the inflow cannula is properly texturized, blood clot (or over pannus formation) have formed resulting in ischemic stroke (also called cerebrovascular accident or CVA).

Example devices disclosed herein operate to avoid or minimize blood stagnation, and inflow cannula malposition around the inflow cannulation site of heart. The ultimate goal is to mitigate the risk of cerebrovascular accidents such as ischemic stroke which can result from these problems. More particularly, various disclosed embodiments function to address possible root causes associated with current LVAD inflow cannula.

In some disclosed examples, protrusion of the cannula tip into the ventricle chamber is minimized or eliminated. Since there is no blood flow obstruction in the left ventricle, blood flow is more of a physiological pattern, and is particularly beneficial for avoiding blood stagnation around the ventricular apex. Since the cannula shows little or no wedge between the cannula wall and myocardium, theoretically there is no chance of wedge thrombus formation. Also, blood contacting surface area in the left ventricle is minimal.

Since the cannula does not extend significantly into the ventricle, there is no tilting tip against the ventricular wall, eliminating or at least reducing the chance of malposition. Disclosed arrangements where the cannula tip does not protrude into the ventricle are also avoid inflow ostium occlusion even after a failing heart is remodeled and the heart size shrinks.

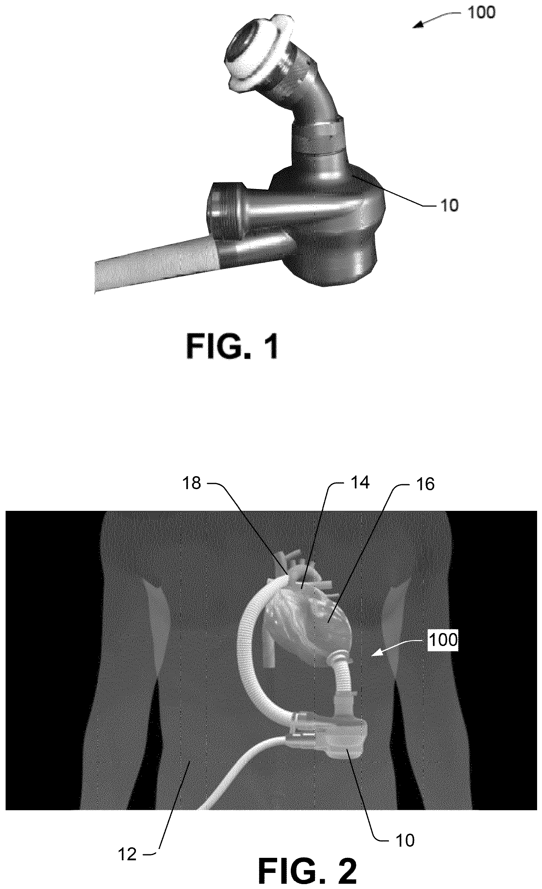

Figure 1 illustrates an example of a cannula system 100 connected to a blood pump 10, and Figure 2 illustrates the blood pump 10 implanted in a human body 12. In the illustrated example, the pump 10 is an LVAD, which is connected to a heart 14 by the cannula system 100. In typical implantation situations, the left ventricle 16 of the heart 14 has been weakened in heart failure and is thus no longer able to sufficiently pump blood from the left ventricle 16 to the aorta 18, which supplies the oxygenated blood to the body. When the patient's heart 12 is no longer able to adequately pump arterial blood to the body, implant of the LVAD 10 may be necessary. The left ventricle 16 of the heart 14 is cored with a circular knife to create a hole at the apex of the left ventricle 16. The LVAD inflow cannula system 100 is sutured into the left ventricle 16 to draw blood from the left ventricle 16 and pump it to the aorta 18.

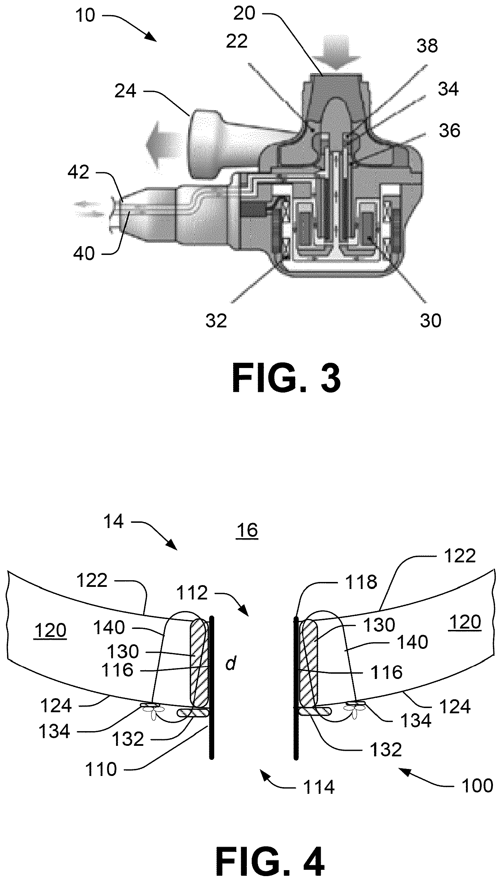

Figure 3 illustrates an example of an implantable blood pump 10, which in some embodiments is an LVAD pump. The pump 10 includes an inlet 20 that is connected to the ventricle 16 via the cannula system 100. An impeller 22 rotates, drawing the blood from the inlet 20 and pumping it to a blood outlet 24. The blood outlet 24 connects to an outflow graft through which blood is returned to the aorta 18. The impeller 22 is connected to a motor rotor 30 which contains permanent magnets in the illustrated example. These magnets in the motor rotor 30 are rotated by a rotating magnetic field created by a motor coil 32 or winding. The blood is restricted from entering the motor rotor 30 by a mechanical seal that includes a rotating seal ring 34 positioned against a non-rotating seat ring 36. A cushion ring 38 is positioned adjacent the seal ring 34 opposite the seat ring 36. The motor coil 32, motor rotor 30, seat ring 36 and seal ring 32 are cooled and lubricated by circulating sterile water that flows in and out of the pump 10 through a water inlet 40 and a water outlet 42, respectively.

The pump 10 is made of titanium, for example, and is powered through a percutaneous cable which transverses the patient's skin and connects to an external battery powered, controller in some embodiments. The blood outlet 24 is connected to the aorta 18 by an outflow graft, which is flexible and made of a sealed polyester material or e-PTFE (polytetrafluoroethylene) in some examples.

Figure 4 is a schematic cross section view illustrating aspects of an embodiment of the cannula system 100. In the illustrated example, the cannula system 100 provides a fluid connection the patient's heart 14. Some examples illustrated herein are described in terms of an LVAD system to provide a fluid connection to the inlet 20 of the pump 10, though the cannula system 100 is applicable to other procedures requiring a fluid connection to a heart. For instance, the cannula system 100 could be used to connect a patient's ventricle to a flow path from the ventricle to the aorta, as in an apicoaortic conduit, for example.

The illustrated cannula system 100 includes a cannula 110 with a first, or connection end 112 that is connected to the myocardium 120 of the heart 14, such that the cannula 110 is in fluid communication with the desired chamber 16 of the heart 14. An opposite end 114 of the cannula 110 connects, for example, to the inlet 20 of the pump 10. When implemented in conjunction with an LVAD, the connection end 112 of the cannula 110 is connected to the apex of the left ventricle of the heart 14. More particularly, the connection end 112 extends through the myocardium 120 such that an inlet 118 of the cannula 110 is in fluid communication with the left ventricle 16 of the heart 14. As shown in Figure 4, inlet 118 of the cannula 110 is in fluid communication with the interior of the heart chamber 16, but it does not extend significantly beyond the endocardium 122 of the heart 14. Some embodiments of the cannula 110, for example, have an inner diameter of about 14mm to 16mm, though the diameter could range to as small as 8mm for pediatric devices to as large as 25mm for implementations with an artificial heart.

A first, or proximal sewing cuff 130 extends around the connection end 112 of the cannula 110, and in the example shown in Figure 4, covers essentially the entire outer surface of the portion of the connection end 112 that extends through the myocardium 120. A second, or distal sewing cuff 132 extends around the connection end 112 adjacent the second end 114 of the cannula 110 at the distal end of the proximal sewing cuff 130. The distal sewing cuff 132 has a greater diameter than the diameter of the proximal sewing cuff 130, such that the sewing cuffs 130, 132 together define a "top hat" configuration. In some examples, the proximal sewing cuff 130 and the distal sewing cuff 132 are two separate components, while in other examples the proximal sewing cuff 130 and the distal sewing cuff 132 are integrally formed. The distal sewing cuff 132 has a diameter that is about twice the diameter of the proximal sewing cuff 130 in some embodiments, and is semi flexible. This allows sutures 140 extending through the distal sewing cuff 132 to "open" the intraventricular shape of the left ventricle 16. In some instances, the distal sewing cuff 132 could be smaller in the case of a pediatric ventricle, where the distal sewing cuff 132 could be the same or nearly the same diameter as the proximal sewing cuff 130. In some instances, the distal sewing cuff 132 diameter could be as large as three times the diameter of the proximal sewing cuff 130 if required to provide support for the myocardium 120 as in the case of a right atrial application with a very thin myocardium 120. One or both of the proximal and distal sewing cuffs 130, 132 may include features to accelerate the healing of the cored myocardium 120.

As will be discussed further below, the apex of the ventricle 16 may be cored to allow the connection end 112 to be inserted into the apex of the ventricle 16. The connection end 112 is secured with an array of sutures 140 around the connection end 112 of the cannula 110. The sutures 140 are inserted through pledgets 134, which are small patches of fabric, usually PTFE or PET, which keep the sutures 140 from cutting the heart muscle. The sutures 140 go through the full thickness of the myocardium 120 and through the endocardium 122 and epicardium 124. The sutures 140 also extend through the proximal and distal sewing cuffs 130, 132, and are tied over the pledgets 134.

In some embodiments, the connection end 112 extends no more than 5 mm beyond the endocardium 122 into the heart chamber 14, and in some examples the connection end 112 extends no more than 0.5 mm beyond the endocardium 122 into the heart chamber 14. The proximal sewing cuff 130 is constructed of sufficient thickness and strength such that a suture 140 can traverse a portion of the interior of the first cuff. In the illustrated example, the proximal sewing cuff is configured such that the sutures 140 are able to extend in a generally axial direction (up and down as shown in Figure 4) from the proximal to the distal end of the proximal cuff 130. This allows the sutures 140 to pull the endocardium flush to the inlet 118. Without the proximal cuff 130, which allows the endocardium 122 to be pulled flush or substantially flush with the inlet 118 around the entirety of the connection end 112, the connection end 112 would have to be longer to insure the inlet 118 is above all of the myocardium 120 and endocardium 122 of the cored area. More precisely, in the illustrated example, the proximal cuff 130 holds the sutures 140 to pull the endocardium 122 flush to the inlet 118, thus allowing the connection end 112 to have minimal insertion into the left ventricle 16.

The sutures 140 going through the endocardium 122 and the proximal sewing cuff 130 result in the myocardium 120 and endocardium 122 being pulled tight against the proximal sewing cuff 130. This protects the blood circulating in the left ventricle 16 from being exposed to the healing myocardium 120, thus reducing the risks of blood clots forming and being pulled into the pump 10. This also provides a seal to prevent or at least reduce blood from leaking along the connection end 112, which would need to be stopped by the distal sewing cuff 132.

The sutures 140 extending through the endocardium 122 and the proximal sewing cuff 130 provide hemostasis at the interior of the left ventricle 16, and a uniform extension of the cannula inlet 118 into the left ventricle 16. With the endocardium 122 fixed relative to the proximal sewing cuff 130, the flexible distal sewing cuff 132 has the effect of pulling the myocardium 120 such that it slightly reshapes the apex of the left ventricle 16 to more of a "U" shape versus a "V" shape. With known pump connections using only a single sewing cuff or other apparatus to secure the cannula tip to the left ventricle apex, when sutures are tightened on the distal cuff, the endocardium can "open up," exacerbating the exposure of the cut myocardium to the left ventricle blood and increasing the likelihood for emboli causing a stroke. With the endocardium 122 fixed relative to the proximal sewing cuff 130, the distal cuff 132 would be flexible to accommodate a thick myocardium 120. The distal cuff 132 could also be movable along the tip 110 to accommodate a thick myocardium 120 while keeping the proximal sewing cuff 130 flush with the endocardium 120.

As mentioned above, the connection end 112 extends into the heart chamber 16 ideally as little as possible, between 0.5 and 5 mm in some examples. However, aspects of the disclosed cannula system 100 generally provides a uniform tip extension and could be applied to longer cannula tips extend farther into the heart chamber 16, for example, 10 to 20 mm.

As also noted previously, the disclosed cannula system 100 is not limited to use associated with the left or right ventricle. In addition to ventricular blood drainage, the cannula system 100 can be applied to right or left atrium drainage. For instance, a left atrial cannulation would be beneficial for diastolic heart failure and acute heart failure caused by myocardial infarction, which develop friable ventricular apex thus not applicable for apical cannulation.

Since there is little or no part of the cannula system 100 that protrudes into the interior of the heart chamber, a hemostatic plug can be safely inserted into the cannula 110 in situations where the patient's heart recovers and the pump 10 is explanted ("cardiac recovery case").

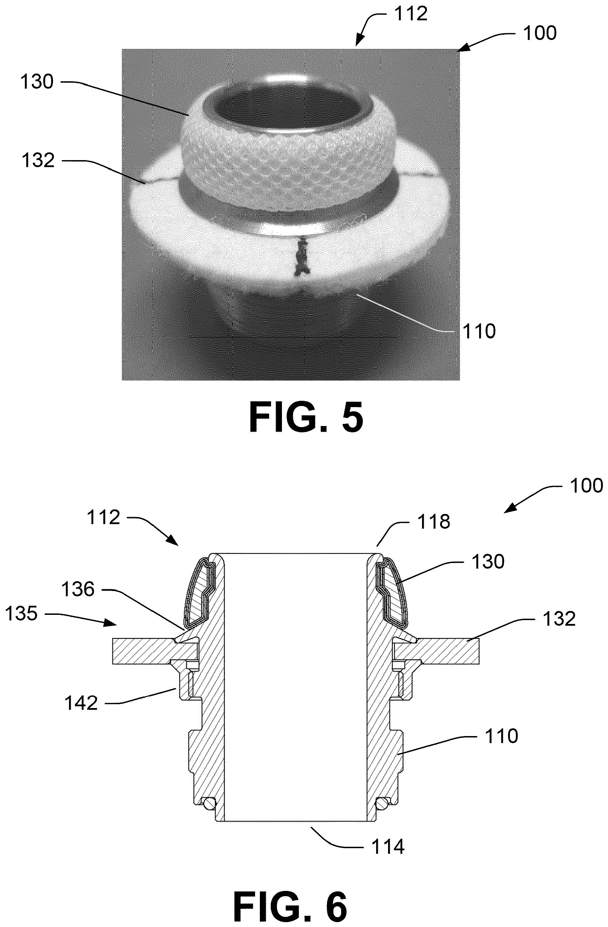

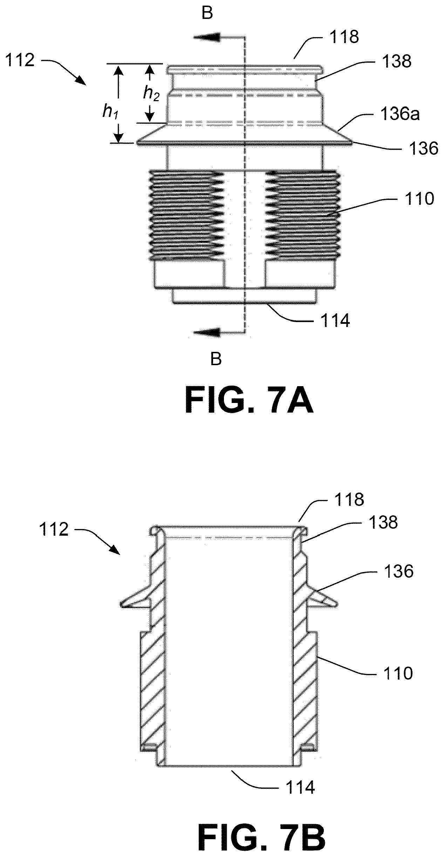

Figure 5 is a perspective view showing an example of the connection end 112 of the cannula system 100, and Figure 6 is a sectional side view of the cannula system 100. Figure 7A is a side view of the cannula 110, and Figure 7B is a side section view of the cannula 110 taken along line B in Figure 7A. The cannula 110 may be fabricated of titanium, for example, and the surface of the cannula 110 may be smooth or textured, and may be uncoated, or coated with a coating such as an antithrombogenic coating.

In the example shown in Figures 5-7, a connector 135 fixes the second cuff 132 to the connection end 112 adjacent the distal end of the proximal cuff 130. In the illustrated embodiment, the connector 135 includes a flange 136 that extends around the connection end 112 of the cannula 110. The flange defines a shoulder 136a that slopes downwardly. The purpose of this flange 136 is to anchor one side of the second cuff 132. When attached to the heart 14, the flange 136 and second cuff 134 are situated on the exterior of the myocardium 120. The body of the cannula 110 is threaded, and the connector 135 further includes a nut 142 that is threaded onto the cannula 110 to sandwich the second sewing cuff 132 between the flange 136 and the nut 142. The threads also facilitate connection of the cannula second end 114 to the inlet 20 of the pump 10.

The illustrated cannula 110 further has a groove 138 extending around the cannula 110 adjacent the inlet 118. In one example, the cylindrical body of the cannula 110 has an inner diameter of 16.00 mm and an outer diameter of 19.5 mm, the flange 136 has a diameter of 27.00 mm, and the groove 138 has a diameter of 18.00 mm. Further, in the illustrated example, the cannula 110 is 30.00 mm high from top to bottom. The connection end 112 has a first height *h₁* of 10.00 mm as measured from the outer edge of the shoulder 136a to the inlet 118, and a second height *h₂* of 7.33 mm as measured from the junction of the top of the shoulder 136a and the exterior surface of the cannula 110 to the inlet 118. The groove 138 is 2.00 mm high and extends into the outer surface of the cannula 0.75 mm.

In the example shown in Figures 5 and 6, the first sewing cuff 130 covers essentially the entire outer surface of the portion of the connection end 112 between the flange 136 and the inlet 118. In other examples, the proximal sewing cuff 130 covers the majority of the outer surface of the connection end 112 between the flange 136 and the inlet 118. According to the invention, the proximal sewing cuff 130 covers at least half of the outer surface of the connection end 112 between the flange 136 and the inlet 118.

The depth (up-and-down dimension as illustrated in Figure 6) of the proximal sewing cuff 130 (and the corresponding portion of the connection end 112) is about 7 mm in some embodiments, but could range from 3 mm for thin, right ventricle application, to 20 mm for patients with an extraordinarily thick myocardium 120. The thickness (side-to-side dimension in Figure 6) of the proximal sewing cuff 130 is between 1 and 3 mm in some examples, and in the embodiment shown in Figures 5 and 6 is 2 mm thick. In general, the thickness of the proximal sewing cuff 130 is sufficient to allow a needle and sutures 140 to pierce the upper most portion of the proximal sewing cuff 130 and traverse through at least a portion of the proximal sewing cuff 130. The proximal sewing cuff 130 may be fabricated with a combination of a felt core with multiple layers of implant grade mesh. The mesh or other implant grade fabric needs to be of sufficient strength to hold the sutures 140 that pull the endocardium 122 flush with the proximal cuff 130. Other variations do not include the felt core. Some implementations employ three layers of mesh, for example. The felt core assists with hemostasis. The felt and mesh composite structure of the proximal sewing cuff 130 provides the structural strength for the sutures 140 coming from the endocardium 122. The mesh allows the anchoring of the pseudo neointima layer that forms from the endocardium 122 to, and over, the mesh. The thickness of the proximal sewing cuff 130 accommodates the suture 140 that enters in the proximal end of the proximal sewing cuff 130 and exits the distal end of the proximal sewing cuff 130. The illustrated proximal sewing cuff 130 has a generally rectangular cross section, though other embodiments define round, trapezoidal, oval, or other shapes to facilitate suture placement.

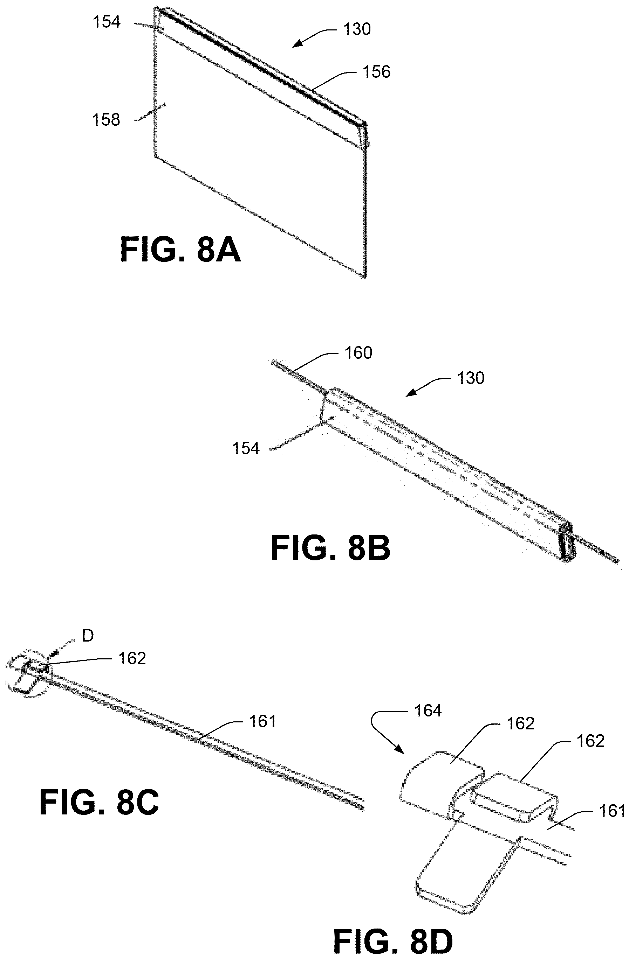

Figures 8A-8D illustrate further details of examples of the proximal sewing cuff 130. In some embodiments, the proximal sewing cuff 130 is secured to the connection end 112 with a fastener that extends around the connection end 112. More particularly, the fastener is received in the groove 138 to attach the proximal sewing cuff 130 to the connection end 112.

Figure 8A shows the proximal sewing cuff 130 prior to its being formed into the shape shown in Figure 8B. In the example shown in Figures 8A and 8B, an implant grade adhesive strip 154 (such as silicone or urethane) is fastened to one side of a felt core 156. The felt core 156 is positioned over a titanium wire 160, and an implant grade mesh 158 is wound about the wire 160 and felt 156 to form the proximal sewing cuff 130 with the felt core 156 surrounded by multiple layers of mesh 158. The titanium wire 160 is pulled into the groove 138 and twisted to secure the proximal sewing cuff 130 to the cannula 110. In other embodiments, the titanium wire 160 is replaced by a titanium band 161, an example of which is shown in Figures 8C and 8D. The felt 156 and mesh 158 are positioned about the titanium band 161 in the same manner as shown in Figures 8A and 8B. One end of the titanium band 161 includes one or more tabs 162 that are bent over to form a channel 164. The proximal sewing cuff 130 formed using the titanium band 164 is fastened to the connection end 112 by situating the titanium band 161 around the connection end 112 in the groove 138. The end of the titanium band 161 opposite the tabs 162 is inserted into the channel 164, and the tabs 162 are crimped to fasten the proximal cuff 130 to the connection end.

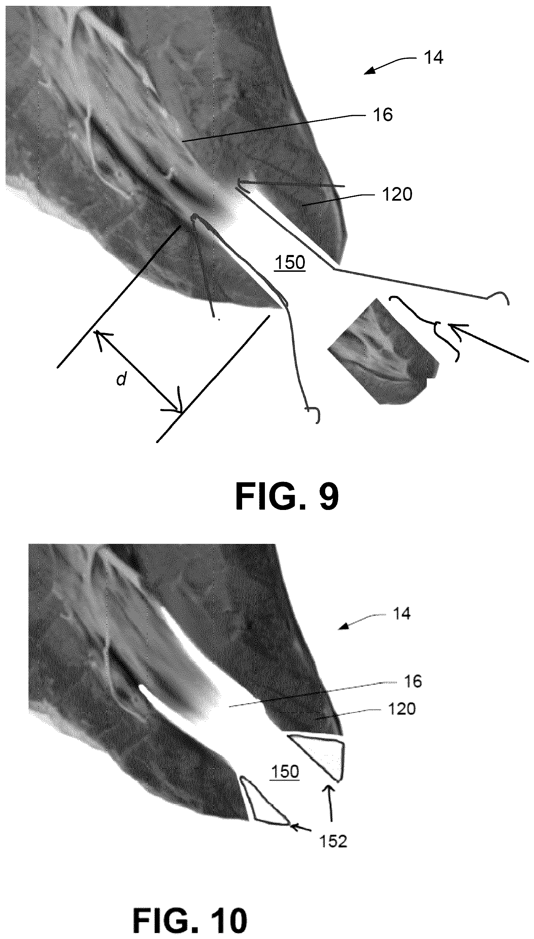

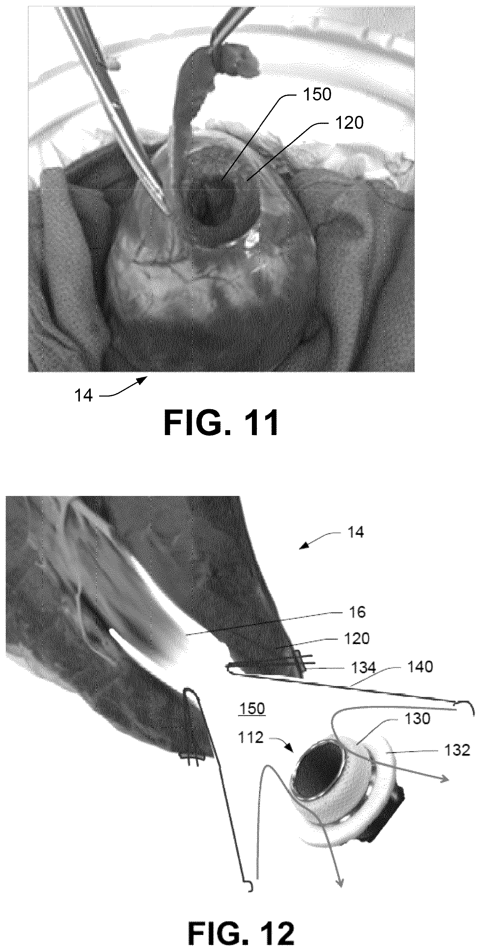

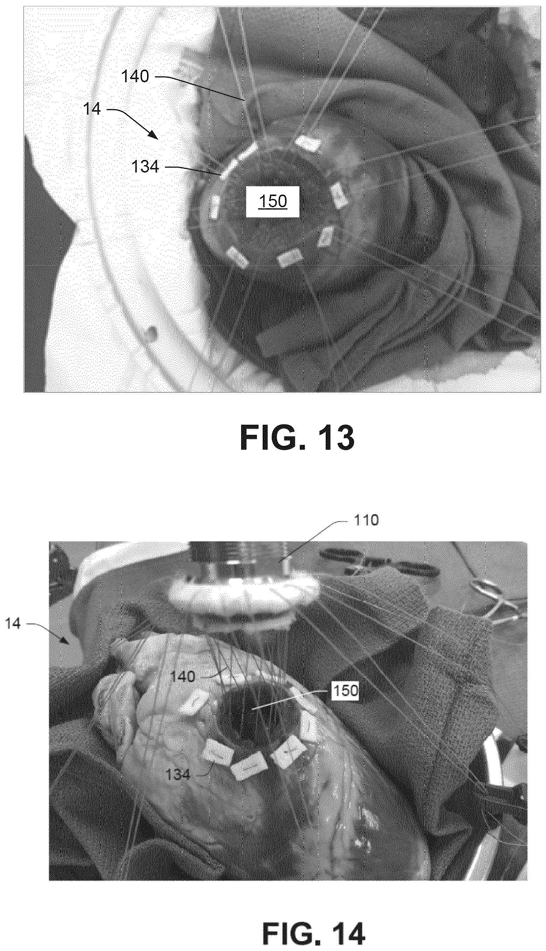

Figures 9-16 illustrate aspects of a surgical procedure for connecting the cannula system 100 to the left ventricle 16 of a heart 14. In Figure 9, the apex of the left ventricle 16 is cored using coring knife or puncher or a similar cutting tool, thus creating an opening 150 extending through the myocardium 120. In Figure 9, the myocardium 120 has a depth *d* that is about the same as the height *h₁* of the connection end 112 of the cannula 110, or is between the heights *h₁* and *h₂*. Accordingly, as noted previously, the connection end 112 extends only minimally into the heart chamber 16. Prior to coring the ventricle 16, the heart is repositioned in the chest so the apex points upwards. Surgical linens may be added to help retract the heart. Figures 10 and 11 illustrate modifying the opening 150, including a wedge cut 152 performed around the apex opening 150 so as to widen the outside of the opening 150, but not the inside, so that the opening 150 defines a shape like a funnel. This wedge cut may be necessary for a thick myocardium so that the endocardium can be pulled flush to the proximal sewing cuff 130. Extra myocardial trabaeculae may further be removed. This also reduces view obstructions, and reduces risks of a myocardial tear.

As shown in Figures 12-14, to attach the connection end 112 of the cannula 110, several braided 2-0 mattress sutures 140 are threaded through the myocardium 120 through the pledgets 134 from outside to inside using, for example, a half circle, 35 mm radius, taper point needle (such as a CV300 TI-CRON needle). In certain examples, eight to twelve sutures 140 are used. The sutures 140 are threaded through the proximal and distal sewing cuffs 130, 132 in one stitch. As noted herein above, the proximal sewing cuff 130 and the distal sewing cuff 132 are configured such that the needle and suture 140 can penetrate the inlet end of the proximal cuff 130, travel through at least a portion of the proximal cuff 130, then pierce the distal cuff 132 in one motion. The CV300 TI-CRON needle mentioned earlier is big enough to allow threading through both the proximal and distal sewing cuffs 130, 132 in one motion. In the illustrated example, the path of the sutures 140 is the epicardium 124, myocardium 120, endocardium 122, proximal sewing cuff 130, and distal sewing cuff 132.

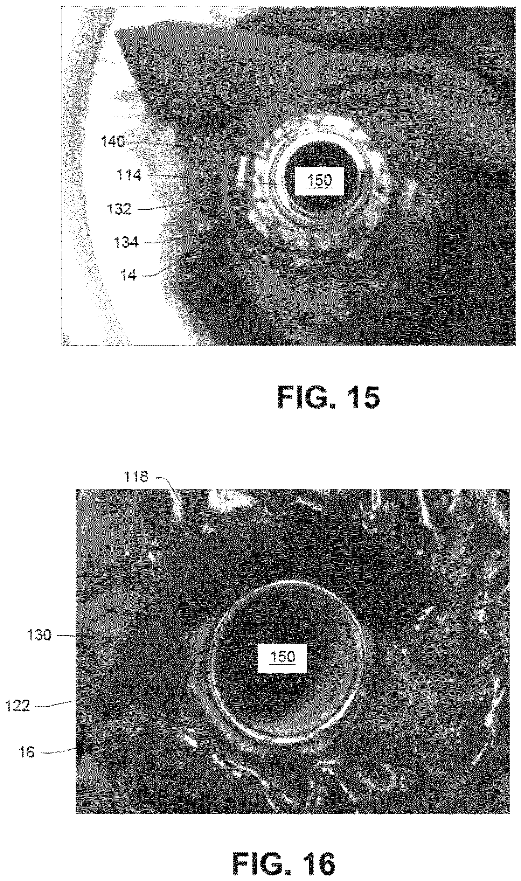

The connection end 112 is parachuted down, with the proximal sewing cuff 130 situated inside the cored myocardium 120, and with the distal sewing cuff 132 resting outside the myocardium 120 against the epicardium 124. The mattress sutures 140 are tied off, and a continuous running suture is provided around distal sewing cuff 132 as shown in Figure 15. Figure 16 shows the inlet 118 of the cannula 110 and a portion of the proximal sewing cuff 130 as viewed from inside the heart chamber 16. As shown in Figure 16, the inlet 118 projects minimally or not at all beyond the endocardium 122 into the heart chamber 16.

## Claims

1. A cannula system (100), comprising:
a cannula (110) defining an inlet (118);
a flange (136) extending around the cannula; and
a first cuff (130) extending around the cannula, **characterized in that** the first cuff covers at least half of an exterior surface of the cannula between the flange and the inlet.

2. The system of claim 1, wherein the first cuff (130) covers a majority of the exterior surface of the cannula (110) between the flange (136) and the inlet (118).

3. The system of claim 1, wherein the cannula (110) is configured to extend into a chamber (16) of a heart (14) not more than 5 mm beyond an endocardium (122) of the heart.

4. The system of claim 1, wherein the cannula (110) is configured to extend into a chamber (16) of a heart (14) not more than 0.5 mm beyond an endocardium (122) of the heart.

5. The system of claim 1, further comprising a second cuff (132) extending around the cannula (110) adjacent the flange (136).

6. The system of claim 5, wherein the second cuff (132) is located between the flange (110) and the inlet (118).

7. The system of claim 5, wherein the second cuff (132) is integrally formed with the first cuff (130).

8. The system of claim 5, wherein the flange (136) is located between the first (130) and second (132) cuffs.

9. The system of claim 5, wherein the first cuff (130) defines a first diameter and the second cuff (132) defines a second diameter greater than the first diameter.

10. The system of claim 1, further comprising a groove (138) extending around the cannula (110) adjacent the inlet (118), and wherein the first cuff (130) includes a fastener (160) received in the groove.

11. The system of claim 1, wherein the first cuff (130) includes a felt core (156) with a plurality of layers of mesh (158).

12. The system of claim 5, wherein
the first cuff has a proximal end adjacent the inlet; and
the second cuff (132) extends around a first end of the cannula (110) adjacent a distal end of the first cuff (130).

## Patentansprüche

1. Kanülensystem (100), umfassend:
eine Kanüle (110), die einen Einlass (118) definiert;
einen Flansch (136), der sich rings um die Kanüle erstreckt; und
eine erste Manschette (130), die sich rings um die Kanüle erstreckt,
**dadurch gekennzeichnet, dass**
die erste Manschette wenigstens die Hälfte einer äußeren Oberfläche der Kanüle zwischen dem Flansch und dem Einlass bedeckt.

2. System nach Anspruch 1, wobei die erste Manschette (130) einen Großteil der äußeren Oberfläche der Kanüle (110) zwischen dem Flansch (136) und dem Einlass (118) bedeckt.

3. System nach Anspruch 1, wobei die Kanüle (110) derart ausgestaltet ist, dass sie sich nicht mehr als 5 mm über eine Innenhaut (122) eines Herzens hinaus in eine Kammer (16) des Herzens (14) erstreckt.

4. System nach Anspruch 1, wobei die Kanüle (110) derart ausgestaltet ist, dass sie sich nicht mehr als 0,5 mm über eine Innenhaut (122) eines Herzens hinaus in eine Kammer (16) des Herzens (14) erstreckt.

5. System nach Anspruch 1, des Weiteren umfassend eine zweite Manschette (132), die sich rings um die Kanüle (110) benachbart zu dem Flansch (136) erstreckt.

6. System nach Anspruch 5, wobei die zweite Manschette (132) zwischen dem Flansch (110) und dem Einlass (118) befindlich ist.

7. System nach Anspruch 5, wobei die zweite Manschette (132) einstückig mit der ersten Manschette (130) ausgebildet ist.

8. System nach Anspruch 5, wobei der Flansch (136) zwischen der ersten Manschette (130) und der zweiten Manschette (132) befindlich ist.

9. System nach Anspruch 5, wobei die erste Manschette (130) einen ersten Durchmesser definiert und die zweite Manschette (132) einen zweiten Durchmesser, der größer als der erste Durchmesser ist, definiert.

10. System nach Anspruch 1, des Weiteren umfassend eine Nut (138), die sich rings um die Kanüle (110) benachbart zu dem Einlass (118) erstreckt und wobei die erste Manschette (130) einen Befestiger (160), der in der Nut aufgenommen ist, beinhaltet.

11. System nach Anspruch 1, wobei die erste Manschette (130) einen Filzkern (156) mit mehreren Netzschichten (158) beinhaltet.

12. System nach Anspruch 5, wobei
die erste Manschette ein proximales Ende benachbart zu dem Einlass aufweist; und
die zweite Manschette (132) sich rings um ein erstes Ende der Kanüle (110) benachbart zu einem distalen Ende der ersten Manschette (130) erstreckt.

## Revendications

1. Système de canule (100), comprenant :
une canule (110) définissant une entrée (118) ;
une bride (136) s'étendant autour de la canule ; et
un premier manchon (130) s'étendant autour de la canule, **caractérisé en ce que** le premier manchon recouvre au moins la moitié d'une surface extérieure de la canule entre la bride et l'entrée.

2. Système selon la revendication 1, dans lequel le premier manchon (130) recouvre une majorité de la surface extérieure de la canule (110) entre la bride (136) et l'entrée (118).

3. Système selon la revendication 1, dans lequel la canule (110) est configurée pour s'étendre dans une chambre (16) d'un coeur (14) pas plus de 5 mm au-delà d'un endocarde (122) du coeur.

4. Système selon la revendication 1, dans lequel la canule (110) est configurée pour s'étendre dans une chambre (16) d'un coeur (14) pas plus de 0,5 mm au-delà d'un endocarde (122) du coeur.

5. Système selon la revendication 1, comprenant en outre un second manchon (132) s'étendant autour de la canule (110) adjacent à la bride (136).

6. Système selon la revendication 5, dans lequel le second manchon (132) est situé entre la bride (110) et l'entrée (118).

7. Système selon la revendication 5, dans lequel le second manchon (132) est formé d'un seul tenant avec le premier manchon (130).

8. Système selon la revendication 5, dans lequel la bride (136) est située entre les premier (130) et second (132) manchons.

9. Système selon la revendication 5, dans lequel le premier manchon (130) définit un premier diamètre et le second manchon (132) définit un second diamètre supérieur au premier diamètre.

10. Système selon la revendication 1, comprenant en outre une rainure (138) s'étendant autour de la canule (110) adjacente à l'entrée (118), et dans lequel le premier manchon (130) comporte un élément de fixation (160) reçu dans la rainure.

11. Système selon la revendication 1, dans lequel le premier manchon (130) comporte une âme en feutre (156) avec une pluralité de couches de maillage (158).

12. Système selon la revendication 5, dans lequel
le premier manchon a une extrémité proximale adjacente à l'entrée ; et
le second manchon (132) s'étend autour d'une première extrémité de la canule (110) adjacente à une extrémité distale du premier manchon (130).
